Europäisches Patentamt

European Patent Office

Office européen des brevets

(19)

(11) Publication number: **0 228 861 B1**

(12) # EUROPEAN PATENT SPECIFICATION

(45) Date of publication of patent specification: **27.11.91**    (51) Int. Cl.5: **A61K 35/74**, C12N 1/20

(21) Application number: **86309810.9**

(22) Date of filing: **16.12.86**

(54) **Agent for reducing the dimethylnitrosoamine level.**

(30) Priority: **19.12.85 JP 284329/85**

(43) Date of publication of application:
**15.07.87 Bulletin 87/29**

(45) Publication of the grant of the patent:
**27.11.91 Bulletin 91/48**

(84) Designated Contracting States:
**CH DE FR GB IT LI NL SE**

(56) References cited:
**EP-A- 0 116 232**
**EP-A- 0 135 820**
**GB-A- 2 072 502**
**US-A- 4 306 024**

**PATENT ABSTRACTS OF JAPAN, vol. 7, no.
19 (C-147)[1164], 25th January 1983; & JP-
A-57 175 124 (KIYOURIN SEIYAKU K.K.)
28-10-1982**

**CHEMICAL ABSTRACTS, vol. 88, no. 5, 30th
January 1978, page 362, abstract no. 35859f,
Columbus, Ohio, US; & JP-A-77 117 494
(MORINAGA MILK INDUSTRY CO., LTD)
01-10-1977**

(73) Proprietor: **KABUSHIKI KAISYA ADVANCE
5-7, Nihonbashi Kobuna-cho
Chuo-ku Tokyo 103(JP)**

(72) Inventor: **Kawai, Yasuo
2-8-12, Mouridai
Atsugi-shi Kanagawa(JP)**
Inventor: **Suegara, Nobuo
3256-6 Kawajiri Shiroyamamachi
Tsukui-gun Kanagawa(JP)**
Inventor: **Okazaki, Suguru
Kopo-Yamaguchi B-203 13-8 Aoba-cho
Sendai Miyagi 980(JP)**

(74) Representative: **Ellis, John Clifford Holgate et
al
MEWBURN ELLIS & CO. 2/3 Cursitor Street
London EC4A 1BQ(GB)**

Rank Xerox (UK) Business Services

## Description

BACKGROUND OF THE INVENTION

1. Field of the Invention

The present invention relates to anti-carcinogenically active products, processes for producing the same, pharmaceutical preparation containing the same, and novel strains of the genera Streptococcus, Bifidobacterium and Lactobacillus suitable for use in the production of the same.

2. Description of the Related Art

In Japan, deaths due to cancer, mostly gastro-intestinal cancer, are increasing, and this disease must be ranked first among geriatric diseases.

As is well-known in the art, several pharmaceutical preparations such as immune activating agents have been proposed as a therapeutical or preventive medicine suitable for cancer. Nevertheless, the number of patients suffering from cancer is increasing, and thus it is clear that the desired purposes of these known medicines are not achieved, from the viewpoint of, for example, pharmacological effects and side-effects, and therefore, there is an increasing need to develop more effective medicines.

SUMMARY OF THE INVENTION

Dimethylnitrosoamine (DMNA) causes cancers of the digestive tract in mammals, and consequently any material which can effectively reduce DMNA in mammals will have anticarcenogenic activity.

It has now been found that both viable cells and dead cells of certain novel strains of microorganism have got the power to effectively reduce DMNA in mammals, and in consequence have anticarcinogenic activity. These strains are:

Streptococcus faecium AD1008 (FERM BP-1212)
Streptococcus faecalis AD9005 (FERM BP-1215)
Streptococcus avium AD2007 (FERM BP-1213)
Bifidobacterium breve AD0055 (FERM BP-1216)
Bifidobacterium adolescentis AD0052 (FERM BP-1217)
Lactobacillus acidophilus AD0006 (FERM BP-1206)
Lactobacillus salivarius AD0009 (FERM BP-1209)

In accordance with the present invention, therefore, we provide a pharmaceutical preparation comprising an amount, effective to reduce the DMNA level, of viable cells, dead cells or a mixture thereof of at least one of the above strains and a pharmaceutically acceptable carrier therefor. These microorganisms derived from so-called gastrointestinal bacteria are substantially nontoxic when orally administered.

The types and morphological characteristics, the screening methods, the preparation methods of strains, and the pharmacological effects of the microorganisms according to the present invention will be explained in detail below.

Microorganisms

The strains of microorganism with which this invention is concerned have been deposited since 13 December 1985 in the Fermentation Research Institute (FRI) in Japan (all the numbers quoted as "FERM-P" in Table 1 refer to the deposition numbers of said institute) and transferred to the Fermentation Research Institute (FRI) (i.e. International Depository Authority under the Budapest Treaty in Japan) as the FERM-BP deposition numbers listed in Table 1 under the Budapest Treaty. Shown also in Table 1 are the corresponding deposition numbers of other strains which are referred to in the description by way of comparison.

Table 1

| Name of strain | Deposition number | |
|---|---|---|
| Streptococcus faecium AD1008 | FERM P-8569 | FERM BP-1212 |
| Streptococcus avium AD2007 | FERM P-8570 | FERM BP-1213 |
| Streptococcus faecalis AD9005 | FERM P-8572 | FERM BP-1215 |
| Bifidobacterium breve AD0055 | FERM P-8573 | FERM BP-1216 |
| Bifidobacterium adolescentis AD0052 | FERM P-8574 | FERM BP-1217 |
| Bifidobacterium longum AD0056 | FERM P-8562 | FERM BP-1205 |
| Lactobacillus acidophilus AD0006 | FERM P-8563 | FERM BP-1206 |
| Lactobacillus fermentum AD0007 | FERM P-8564 | FERM BP-1207 |
| Lactobacillus salivarius AD0009 | FERM P-8566 | FERM BP-1209 |
| Lactobacillus brevis AD0011 | FERM P-8568 | FERM BP-1211 |

General Microbiological Characteristics

The general microorganisms of the present invention are the same as those of known microorganisms belonging to the identical class. That is, the general microbiological characteristics, cultivation methods and other properties correspond to those described in the following articles:

1) Bergey's Manual of Determinative Bacteriology, 8th ed., 490-676 (1974)
2) Int. J. Syst.Bact. 16, 114 (1966)
3) Poupard, J.A., Husain, I. and Norris, R.F.: Bacteriol.Rev.37, 136-165 (1973)
4) Mituoka, T., Jpn. J. Bacteriol.24, 261-280 (1969)

The typical microbiological characteristics of the above-exemplified strains according to the present invention are summarized in Table 2 through Table 4.

## Table 2

| Characteristics | S. faecium AD1008 | S. faecalis AD9005 | S. avium AD2007 |
|---|---|---|---|
| Gram stain | + | + | + |
| Catalase | − | + | − |
| Growth at 10°C | + | + | + |
| Growth at 45°C | + | + | + |
| Growth at pH 9.6 | + | + | + |
| Growth at 6.5% NaCl | + | + | + |
| 40% bile acid tolerance | + | + | + |
| Growth in 1/4000 tellurite | − | + | − |
| Growth in 0.1% methylene blue milk | + | + | − |
| Fermentation of | | | |
| Arabinose | + | − | + |
| Glycerol | − | + | + |
| Raffinose | + | + | + |
| Sorbitol | − | + | + |
| Hydrolysis of esculin | + | + | + |
| Hydrolysis of arginine | + | + | − |

+: positive, −: negative,

## Table 3

| Characteristics | B. adolescentis AD0052 | B. breve AD0055 |
|---|---|---|
| Gram stain | + | + |
| Gas production from glucose | − | − |
| Growth at below 15°C | − | − |
| Growth at 45°C | − | − |
| Catalase | − | − |
| Fermentation of | | |
| Arabinose | + | − |
| Xylose | + | − |
| Ribose | + | + |
| Mannose | +S/+ [*1] | − |
| Fructose | + | |
| Sucrose | + | − |
| Maltose | + | |
| Lactose | + | + |
| Trehalose | V [*4] | − |
| Melibiose | + | V |
| Raffinose | + | + |
| Melezitose | −/+S [*2] | V |
| Dextrin | V | |
| Starch | V | V |
| Glycogen | V | |
| Inulin | V | − |
| Mannitol | V | + |
| Sorbitol | V | + |
| Inositol | − | |
| Esculin | + | +S |
| Amygdalin | + | +S |
| Cellobiose | +S [*3] | +S |
| Salicin | + | V |
| α-methyl glucoside | + | |

*1) Slowly reacted, rarely negative
*2) Negative, rarely and slowly reacted as positive
*3) S: Slowly reacted
*4) V: Variable

## Table 4

| Characteristics | L. acidophilus AD0006 |
|---|---|
| Gram stain | + |
| Gas production from glucose | − |
| Growth at 15 °C | − |
| Growth at 45 °C | + |
| Fermentation of | |
|   Arabinose | − |
|   Xylose | − |
|   Rhamnose | − |
|   Ribose | − |
|   Mannose | + |
|   Fructose | + |
|   Galactose | + |
|   Sucrose | + |
|   Maltose | + |
|   Cellobiose | + |
|   Lactose | + |
|   Trehalose | − |
|   Melibiose | + |
|   Raffinose | − |
|   Melezitose | − |
|   Dextrin, Starch | V [*1] |
|   Mannitol | − |
|   Sorbitol | − |
|   Esculin | + |
|   Salicin | − |
|   Amygdalin | + |

*1) V: Variable

The cultivation of these microorganisms is conventional, as mentioned above. For example, the bacterial cells of Streptococcus and Lactobacillus can be collected by stationarily cultivating in a Rogosa broth medium having the following composition under an aerobical condition or an anaerobical condition, and in the case of Bifidobacterium, in a GAM broth medium under an anaerobical condition, and can be harvested by centrifugation of the culture.

## Composition of Rogosa broth medium

| | |
|---|---|
| Trypticase | 10 g |
| Yeast extract | 5 g |
| Tryptose | 3 g |
| $K_2HPO_4$ | 3 g |
| $KH_2PO_4$ | 3 g |
| Sodium acetate[1] | 1 g |
| Triammonium citrate | 2 g |
| Tween 80[2] | 1 g |
| Glucose | 20 g |
| L-cysteine hydrochloride | 0.2 g |
| Salt solution[3] | 5 ml |
| Distilled water | to 1 liter |

(pH 7, heat-sterilization at 121°C for 15 minutes)

[1]: Unnecessary for Streptococcus
[2]: Polyoxyethylene (20) sorbitan monooleate available from Atlas Powder Co.
[3]: 

| | |
|---|---|
| $MgSO_4-7H_2O$ | 11.5 g |
| $FeSO_4-7H_2O$ | 0.68 g |
| $MnSO_4-2H_2O$ | 2.4 g |
| Distilled water | 100 ml |

## Composition of GAM broth medium

GAM broth "Nissui Seiyaku K.K." code 05422

|  | In 59.0 g |
| --- | --- |
| Peptone | 10.0 g |
| Soybean peptone | 3.0 g |
| Proteose peptone | 10.0 g |
| Digested serum powder | 13.0 g |
| Yeast extract | 5.0 g |
| Meat extract | 2.2 g |
| Liver extract powder | 1.2 g |
| Glucose | 3.0 g |
| $KH_2PO_4$ | 2.5 g |
| NaCl | 3.0 g |
| Soluble starch | 5.0 g |
| L-cysteine hydrochloride | 0.3 g |
| Sodium thioglycolate | 0.3 g |
| Distilled water | to 1 liter |
|  | (pH $7.3\pm0.1$) |

(heat-sterilization at 121°C for 15 minutes)

The resultant cells can be directly used as a pharmaceutical agent in the form of viable cells or dead cells obtained by, for example, heat-treatment, or in the form of the cells destroyed by, for example, ultrasonic treatment. The term "dead cells" used herein means the entire portions or partial portions of the above-mentioned destroyed cells.

### Screening Methods

The screening of the microorganisms can be carried out by a method of Mituoka, T., (1971), J. Jap. Assoc. Infect. Dis. 45, 406.

That is, as mentioned in this literature, the feces obtained from healthy adults were diluted 10 fold in the following diluent and smeared on KMN agar (Vander Wiel-Korstanje, J. A. A., and K. C. Winkler; J. Med. Microbiol. 8, 491 (1975)) for Streptococcus, on LBS agar (BBL) for Lactobacillus, and on MPN agar (Tanaka, R. et al. (1980) Appl. Environ. Microbiol. 40, 866-869) for Bifidobacterium, respectively (the compositions of the broth media are as shown below), and cultivated at 37°C for 48 to 120 hours. The formed colonies were counted and were randomly isolated. The colony type and catalase activity, Gram stain, and the shape of cell were determined and the isolates were identified and classified by examining physiological, biochemical, and serological properties.

## Composition of diluent

| | |
|---|---|
| $KH_2PO_4$ | 4.5 g |
| $Na_2HPO_4$ | 6.0 g |
| L-cysteine hydrochloride | 0.5 g |
| Tween 80[*1] | 0.5 g |
| Agar | 1.0 g |
| Distilled water | 1000 ml |

*1: Polyoxyethylene (20) sorbitan monooleate available from Atlas Powder Co.

## Composition of KMN agar

| | |
|---|---|
| $NaN_3$ | 0.2 g |
| Tryptose | 15.0 g |
| Meat extract | 3.0 g |
| NaCl | 5.0 g |
| Skim milk | 16.0 g |
| Neutral red | 40 mg |
| Kanamycin | 24 mg |
| Agar | 18 g |
| Distilled water | to 1000 ml |
| pH = 7.0 | |

## <u>Composition of LBS agar</u>

| | |
|---|---:|
| Tryptose | .10 g |
| Meat extract | 5 g |
| $KH_2PO_4$ | 6 g |
| Triammonium citrate | 2 g |
| Glucose | 20 g |
| Anhydrous sodium acetate | 15 g |
| Tween 80[1] | 1 g |
| $MgSO_4 \cdot 7H_2O$ | 575 mg |
| $MnSO_4 \cdot 2H_2O$ | 120 mg |
| $FeSO_4 \cdot 2H_2O$ | 34 mg |
| Agar | 15 g |
| Distilled water | 1000 ml |
| pH = 5.5 | |

[1]: Polyoxyethylene (20) sorbitan monooleate available from Atlas Powder Co.

## Composition of MPN agar

| | |
|---|---|
| Lactose | · 2 g |
| $(NH_4)_2SO_4$ | 0.5 g |
| $K_2HPO_4$ | 0.1 g |
| $MgSO_4 \cdot 7H_2O$ | 20 mg |
| $FeSO_4 \cdot 7H_2O$ | 1 mg |
| $MnSO_4 \cdot 2H_2O$ | 0.8 mg |
| NaCl | 1 mg |
| 0.1% Resazurin | 0.1 ml |
| Biotin | 0.01 mg |
| Calcium pantothenate | 0.2 mg |
| Riboflavin | 0.1 mg |
| Adenine | 0.1 mg |
| Guanine | 0.1 mg |
| Xanthine | 0.1 mg |
| Uracil | 0.1 mg |
| Tween 80[*1] | 0.1 g |
| 10% Pyruvic acid | 0.1 ml |
| 8% $Na_2CO_3$ | 5.0 ml |
| 3% L-cysteine hydrochloride | 1 ml |
| Nalidixic acid | 10 mg |
| 1.6% Bromcresol purple | 0.1 ml |
| Agar | 2 g |
| Distilled water | to 100 ml |
| pH=6.8 | |

*1: Polyoxyethylene (20) sorbitan monooleate available from Atlas Powder Co.

Preparation of Bacterial Cells

The viable cells and dead cells of the microorganisms used in the present invention, suitable for use as an anticarcinogenic and antimutagenic agent, are typically prepared as follows:

1. Preparation Example of the Viable Cells

Each strain of the above-mentioned microorganisms is inoculated into 5 liters of the above-mentioned Rogosa broth medium (in the case of Lactobacillus and Streptococcus) or GAM broth medium (in the case of Bifidobacterium), and the former is then stationarily cultivated under an aerobic or an anaerobic condition, and the latter under an anaerobic condition, each at 37°C for 8 hours to yield the subsequent culture broth containing $10^9$/ml of the viable cells. The microorganisms are harvested by continuous centrifugation at 12,000 rpm. The bacterial cells are washed with physiological saline, and are then suspended in physiological saline to obtain 50 ml of the cell suspension containing $10^{11}$ cells/ml.

2. Preparation Example of the Dead Cells

The viable cells obtained as mentioned in the above Example are further washed twice with physiological saline (0.85% NaCl solution) and are then suspended in the same solution. Fifty ml of the cell suspension thus obtained ($10^{11}$ cells/ml) was heated at 115°C for 10 minutes to form the desired cell suspension containing the dead cells.

Pharmacological Actions

1. Pharmacological Effects

a) As shown in each example hereinbelow, the bacterial cell products obtained from the above-mentioned microorganisms of the present invention can make an extremely effective reduction of dimethylnitrosoamine (DMNA) level in mammals. Accordingly, the bacterial cell products according to the present invention are useful as a preventive medicine for cancer of the digestive tracts (especially stomach and hepatic cancer).

b) The pharmaceutical composition according to the present invention can be generally applied in a dosage of 0.1 mg/kg to 10 g/kg body weight, more desirably 1 mg/kg to 1 g/kg body weight, by, for example, an oral or iv. administration. The pharmaceutical composition according to the present invention can be in the form of, for example, suspensions in physiological saline solutions, powder prepared by, for example, freeze-drying, granules, tablets, and capsules. The pharmaceutical composition according to the present invention can be optionally prepared by using conventional appropriate carriers, bulk fillers, diluents, and so on.

2. Acute Toxicity

As shown in the examples hereinbelow, the $LD_{50}$ of the viable cells according to the present invention is 3.9 to 7.2 mg/mouse (intraperitoneal administration) and that of the dead cells according to the present invention is more than 50 mg/mouse (intraperitoneal administration).

Both the viable and dead cells according to the present invention are substantially nontoxic on oral administration.

EXAMPLES

The present invention will now be further shown by, the following examples.

Example 1

The heat-treated dead cell suspension (including 8 mg of wet cells/each tube) of S. faecium AD1008, S. faecalis AD9005, or S. avium AD2007 was prepared according to the above-mentioned Preparation Example of dead cells. To each suspension, 80 μg of DMNA/each tube was added. The mixture was incubated at 37°C for 1 hour and centrifuged at 3500 rpm for 15 minutes. The absorbance at 225 nm of the supernatant of the centrifuged mixture was measured, and the reduction amount and rates (%) of DMNA by the addition of the heat-treated dead bacterial cell suspension were calculated. One hundred percent of the reduction rate was of the controls to which a sample of the cell suspension was not added.

## Table 5

| Strains | Reduction amount of DMNA (μg) | Reduction rate of DMNA (%) |
|---|---|---|
| S. faecium AD1008 | 53.4 | 66.8 |
| S. faecalis AD9005 | 37.9 | 47.1 |
| S. avium AD2007 | 36.8 | 46.0 |

12

Values similar to those in Table 5 can be obtained using the same wet weight of the viable cell suspension of the same respective bacterial strain.

Example 2

The reduction amount (μg) and rates (%) of DMNA by the heat-treated deal cell suspensions of B. adolescentis AD0052, B. breve AD0055, and B. longum AD0056 were measured by the methods shown in Example 1. The results are shown in Table 6.

## Table 6

| Strains | Reduction amount of DMNA (μg) | Reduction rate of DMNA (%) |
|---|---|---|
| B. adolescentis AD0052 | 57.6 | 72.0 |
| B. breve AD0055 | 43.4 | 54.3 |
| B. longum AD0056 | 40.8 | 50.1 |

Values similar to those in Table 6 can be obtained using the same wet weight of the viable cell suspension of the same respective bacterial strain.

Example 3

The reduction amount (μg) and rates (%) of DMNA by the heat-treated dead cell suspensions of L. acidophilus AD0006, L. fermentum AD0007, L. salivarius AD0009 and L. brevis AD0011 were measured by the methods shown in Example 1. The results are shown in Table 7.

## Table 7

| Strains | Reduction amount of DMNA (μg) | Reduction rate of DMNA (%) |
|---|---|---|
| L. acidophilus AD0006 | 28.2 | 35.2 |
| L. fermentum AD0007 | 24.2 | 30.3 |
| L. salivarius AD0009 | 27.3 | 34.1 |
| L. brevis AD0011 | 24.6 | 30.8 |

Values similar to those in Table 7 can be obtained using the same wet weight of the viable cell suspension of the same respective bacterial strain.

Example 4

Viable cells of bacteria prepared according to the above-mentioned Preparation Example of viable cells

EP 0 228 861 B1

were intraperitoneally administered to ICR mice (6 week-old males, average body weight of 31.0 ± 0.5 g) in the form of 0.5 ml of a bacterial suspension containing 10, and 0.1 mg of dry cells per mouse (10 mice in each group). Thus, the thanatobiologic observation of the mice was carried out for 14 days.

The $LD_{50}$ values (viable cell weight/mouse) calculated according to a Behrens-Kärber method are shown in Table 8.

In the case of the dead cells of the above-mentioned strains according to the present invention, the $LD_{50}$ values corresponded to more than 50 mg/mouse (intraperitoneal dosage). Both the viable and dead cells of the above-mentioned strains according to the present invention were nontoxic in the case of oral administration.

| Table 8 | |
| --- | --- |
| Strains | $LD_{50}$ (viable cell weight/mouse) (mg) |
| S. faecium AD1008 | 7.1 |
| S. avium AD2007 | 7.2 |
| B. adolescentis AD0052 | 4.4 |
| B. longum AD0056 | 3.9 |
| L. acidophilus AD0006 | 6.6 |

Example 5 (Pharmaceutical preparations)

1. A 50 mg amount (corresponding to $5 \times 10^{10}$ cells) of freeze-dried powder of B. adolescentis AD0052 viable cells or dead cells prepared according to the above-mentioned Preparation Example of viable or dead cells was uniformly mixed with 950 mg of purified starch powder, and the tablets then formed for oral administration. Each tablet corresponds to a dosage of $10^9$ cells/Kg body weight for a human adult having a body weight of 50 kg.

2. A tablet obtained by mixing 500 mg of the above-mentioned freeze-dried powder with 500 mg of purified starch powder and pressing the mixture corresponds to a dosage of $10^{10}$ cells/kg body weight.

Thus, the cell products of the present invention can be converted into the desired dosage form having a predetermined activity by mixing with pharmaceutically acceptable carriers based on the above-mentioned standard dosage.

## Claims

1. A pharmaceutical preparation for reducing the dimethylnitrosoamine level in mammals comprising an amount, effective to reduce the DMNA level, of viable cells, dead cells or a mixture thereof of at least one of the following strains
   Streptococcus faecium AD1008 (FERM BP-1212)
   Streptococcus faecalis AD9005 (FERM BP-1215)
   Streptococcus avium AD2007 (FERM BP-1213)
   Bifidobacterium breve AD0055 (FERM BP-1216)
   Bifidobacterium adolescentis AD0052 (FERM BP-1217)
   Lactobacillus acidophilus AD0006 (FERM BP-1206)
   Lactobacillus salivarius AD0009 (FERM BP-1209)
   and a pharmaceutically acceptable carrier therefor.

## Revendications

1. Préparation pharmaceutique propre à abaisser le taux de diméthylnitrosoamine chez les mammifères, comprenant une quantité, efficace pour abaisser le taux de DMNA, de cellules viables, de cellules mortes ou d'un mélange de cellules viables et mortes de l'une au moins des souches suivantes
   Streptococcus faecium AD1008 (FERM BP-1212)
   Streptococcus faecalis AD9005 (FERM BP-1215)
   Streptococcus avium AD2007 (FERM BP-1213)
   Bifidobacterium breve AD0055 (FERM BP-1216)

14

*Bifidobacterium adolescentis* AD 0052 (FERM BP-1217)
*Lactobacillus acidophilus* AD0006 (FERM BP-1206)
*Lactobacillus salivarius* AD0009 (FERM BP-1209)
et un excipient pour ces cellules, acceptable sur le plan pharmaceutique.

**Patentansprüche**

1. Pharmazeutisches Produkt zur Verminderung des Dimethylnitrosamines (DMNA)-Blutspiegels in Säugetieren, enthaltend eine zur Verminderung des DMNA-Blutspiegels wirksame Menge von lebensfähigen Zellen, toten Zellen oder einem Gemisch hiervon von mindestens einem der folgenden Stämme:

   Streptococcus faecium AD1008 (FERM BP-1212)
   Streptococcus faecalis AD9005 (FERM BP-1215)
   Streptococcus avium AD 2007 (FERM BP-1213)
   Bifidobacterium breve AD0055 (FERM BP-1216)
   Bifidobacterium adolescentis AD0052 (FERM BP-1217)
   Lactobacillus acidophilus AD0006 (FERM BP-1206)
   Lactobacillus salivarius AD0009 (FERM BP-1209)

   und einen pharmazeutisch annehmbaren Träger hierfür.